# EUROPEAN PATENT SPECIFICATION

(11) **EP 1 549 357 B1**
(45) Date of publication and mention of the grant of the patent: **30.04.2008**
(21) Application number: 03783419.9
(22) Date of filing: 13.11.2003
(51) Int. Cl.: H01L 21/00, B01D 53/66

(54) **WORKPIECE PROCESSING SYSTEM**
ARBEITSSTÜCK-BEARBEITUNGSSYSTEM
SYSTEME DE TRAITEMENT DE PIECES A TRAVAILLER

(30) Priority: 10.12.2002 US 315609; 10.07.2003 US 486771 P
(43) Date of publication of application: 06.07.2005
(73) Proprietor: SEMITOOL, INC., Kalispell, Montana 59901 (US)
(72) Inventor: BREESE, Ronald, G., Whitefish, MT 59937 (US); SCRANTON, Dana, R., Kalispell, MT 59901 (US); BERGMAN, Eric, J., Kalispell, MT 59901 (US); BARTKOSKI, Michael, E., Col. Falls, MT 55912 (US); GROVE, Coby, S., Whitefish, MT 59937 (US)
(74) Representative: Viering, Jentschura & Partner
(86) International application number: PCT/US2003/036283
(87) International publication number: WO 2004/052411

(56) References cited:
- US-A- 6 035 871
- US-A- 6 143 081
- US-A1- 2002 045 008
- US-B1- 6 224 934
- US-B1- 6 273 108
- PATENT ABSTRACTS OF JAPAN vol. 2003, no. 04, 2 April 2003 (2003-04-02) -& JP 2002 353184 A (TOKYO ELECTRON LTD), 6 December 2002 (2002-12-06)
- PATENT ABSTRACTS OF JAPAN vol. 2000, no. 04, 31 August 2000 (2000-08-31) -& JP 2000 024456 A (TOKYO ELECTRON LTD), 25 January 2000 (2000-01-25)

## Description

### BACKGROUND OF THE INVENTION

The invention relates to surface preparation, cleaning, rinsing and drying of workpieces, such as semiconductor wafers, flat panel displays, glass masks, rigid disk or optical media, thin film heads, or other articles or workpieces formed from a substrate on which microelectronic circuits, data storage elements or layers, or micro-mechanical elements may be formed. These and similar articles are collectively referred to here as a "wafer" or "workpiece."

The semiconductor manufacturing industry is constantly seeking to improve the processes used to manufacture microelectronic circuits and components, such as the manufacture of integrated circuits from wafers. The objectives of many of these improved processes are decreasing the amount of time required to process a wafer to form the desired integrated circuits; increasing the yield of usable integrated circuits per wafer by, for example, decreasing contamination of the wafer during processing; reducing the number of steps required to create the desired integrated circuits; and reducing the costs of manufacture.

In the processing of wafers, it is often necessary to subject one or both sides of the wafer to a fluid in either liquid, vapor or gaseous form. Such fluids are used to, for example, etch the wafer surface, clean the wafer surface, dry the wafer surface, passivate the wafer surface, deposit films on the wafer surface, etc.

Various systems and methods have been used for carrying out these manufacturing processes. For example, manual or automated wet benches have long been used for various manufacturing process steps. Wet benches typically have a row of immersion tanks, and a mechanism for sequentially immersing a batch a workpieces into each tank. However, these systems have several disadvantages. These disadvantages include relatively large consumption of process chemicals and water, e.g., 30-35 liters for each wet bench tank, with a bath life of for example 2-4 hours. This consumption of process chemicals increases manufacturing costs, which ultimately increases the cost of the final product, such as e.g., computers, cell phones, and virtually all types of consumer, industrial, commercial and military electronic products.

Many chemistries used in processing, such as HF, HCl, H₂SO₄, and H₂O₂, are toxic, expensive, and/or difficult to handle and dispose of. As a result, complex draining, recycling, and removal systems are often required for effectively handling and disposing of these used processing chemistries. Furthermore, even when proper disposal procedures are followed, there is still a potential for the used processing chemistries to have a negative environmental impact. Accordingly, there is a need for processing machines and methods having less reliance on these types of chemistries.

Reducing consumption of water is also beneficial, especially in areas where clean water is becoming increasingly scarce. Disposing of waste water from manufacturing operations, in environmentally friendly ways, can often be difficult or costly. Accordingly, reducing water consumption in the manufacturing process is important.

Wafers are processed in clean rooms, to reduce potential for contamination resulting in defects in the end products, such as microelectronic or micromechanical devices. Clean rooms are costly and time consuming to build and maintain. Wafer or workpiece processing machines now in use, such as wet benches, often require a large amount of clean room space. This results in higher manufacturing costs and other disadvantages. Accordingly, there is a need for more compact processing machines, which require less clean room space while maintaining or improving on processing speed or throughput.

Overall processing times for large batches of workpieces in existing processing systems are often relatively slow. Wet bench processing can typically take 45 minutes. In some systems, the workpieces may be moved between several processing stations, during a single processing phase or step. This slows total production time. Alternatively, with some processing systems, including spray systems or vapor deposition systems, only a small number of workpieces can be processed at a given time, and/or only a limited number of processing machines or systems can be fit within the clean room. Accordingly, there is a need for workpiece processing systems that are compact but capable of processing large batches of workpieces in a relatively short amount of time.

For certain processing steps, ozone is introduced into the processing chamber. Ozone is typically introduced with water, sometimes containing dilute amounts of chemical. The ozone is then removed from the processing chamber (optionally along with other chemicals, such as acid vapors) and then exhausted to the atmosphere.

However, ozone is a highly chemically reactive gas. In high concentrations, it can become toxic to humans. The exhaust mixture of ozone can therefore be both toxic, and highly corrosive. Consequently, handling the gas exhaust from a processing chamber generally requires special procedures. For example, components such as ducts, etc. generally must be made of PVDF or other plastics which resist corrosion. Leak detectors may also be employed to detect any leaks in the pipes or ducts carrying the exhaust gases from the processing chamber to the outside.

US 2002/0045008 A1 discloses a substrate processing apparatus and a substrate processing method for processing a substrate-to-be-processed held in a processing vessel with ozone gas fed to the substrate, the apparatus comprising: an ozone gas feed system for feeding the ozone gas into the processing vessel; interior exhaust means for discharging an interior atmosphere in the processing vessel; periphery exhaust means for discharging a peripheral atmosphere around the processing vessel; an ozone killer for removing ozone from the interior atmosphere discharged from the interior of the processing vessel.

JP 2002 353184 A discloses substrate processing method and apparatus, wherein after supplying an ozone gas and steam into the processing container and processing a semiconductor wafer, air is supplied from an air supply source connected to an ozone gas supply pipeline for supplying the ozone gas into the processing container, and ozone gas atmosphere inside the processing container is replaced with air atmosphere.

US-A-6 035 871 discloses an apparatus for producing semiconductors, comprising a place in which there is provided a cleaning apparatus for cleaning objects, said cleaning apparatus including a gas generating device for generating an ozone gas and a hydrogen gas, a gas dissolving device for dissolving said ozone gas and said hydrogen gas respectively into pure water to produce ozonized water and hydrogenated water, and a cleaning device for spraying said ozonized water and said hydrogenated water onto the objects.

Ozone converters or destructors have been used to convert ozone in an exhaust flow into oxygen. These converters or destructors typically use catalysts, such as maganese dioxide. The catalyst, however, lose efficiency or ability to catalyze, when they become saturated with condensation. It is also important to prevent stray catalyst particles from moving into the processing chamber, where they can cause contamination.

Accordingly, it is a further object of the invention to provide an improved ozone destructor for destroying ozone, by converting ozone into oxygen.

### BRIEF STATEMENT OF THE INVENTION

Processing systems or machines, and methods, have now been invented to overcome the disadvantages described above. These new systems and methods provide for rapid and efficient wafer processing, and at a reduced cost. In addition, these systems and methods avoid the need for using large amounts of various costly or toxic chemistries. The invention therefore provides for significant advances in the technology of manufacturing semiconductor wafers and similar workpieces. The invention is directed to a system for processing workpieces preferably in a standalone processing apparatus having vertically stacked modules. The vertically stacked modules advantageously contain the system components used in processing the workpieces. To this end, the invention provides a system according to claim 1, and provides a method according to claim 5. Further embodiments of the invention are described in the dependent claims. The system is highly compact and requires a minimum amount of floor space in a clean room. This reduces both initial and follow on operating costs, and allows for higher throughput or manufacturing speed, via more systems within the clean room. In comparison to a typical wet bench system which may require 15 square meters of clean room floor space, the present system occupies less than I or 2 square meters, while having an increased throughput. Moreover, the present system uses only a small fraction of the volume of process chemistries required by wet bench systems. In addition, the initial cost of the present system is less than wet bench systems.

Ozone gas used in processing in the processing chamber is exhausted to the ozone destructor where it is converted to oxygen. This ozone conversion is performed within the system. The potential for release of toxic ozone gas is reduced. The ozone destructor advantageously uses catalyst to convert ozone to oxygen. Saturation of the catalyst with condensation is reduced or avoided by novel design and arrangement of the ozone destructor.

The invention resides as well in sub-combinations of the features described and in the individual components.

### BRIEF DESCRIPTION OF THE DRAWINGS

In the drawings, wherein the same reference number denotes the same element throughout the several views:

Fig. 1 is a front perspective view of a workpiece processing system according to a preferred embodiment, with various covers removed for purpose of illustration.

Fig. 2 is rear perspective view of the processing system of Fig. 1.

Fig. 3 is a perspective view of a processing chamber assembly according to a preferred embodiment.

Fig. 4 is a perspective view of a rotor that may be used in the processing chamber assembly of Fig. 3.

Fig. 5 is a schematic diagram of a preferred processing method.

Fig. 6 is a schematic diagram of an alternative preferred processing method.

Figure 7 is an exploded perspective view of the ozone destructor shown in Figures 1 and 2.

Figure 8 is a section view of a preferred ozone destructor as shown in Figures 5-6.

Figure 9 is a perspective view of an automated processing system including the ozone destructor shown in Figs 7 and 8.

### DETAILED DESCRIPTION OF THE DRAWINGS

The terms workpiece, wafer, or semiconductor wafer, as used here, mean any flat media, including semiconductor wafers and other substrates or wafers, glass, mask, and memory media, MEMS substrates, or any other workpiece having microelectronic, micro-mechanical, or micro electro-mechanical devices.

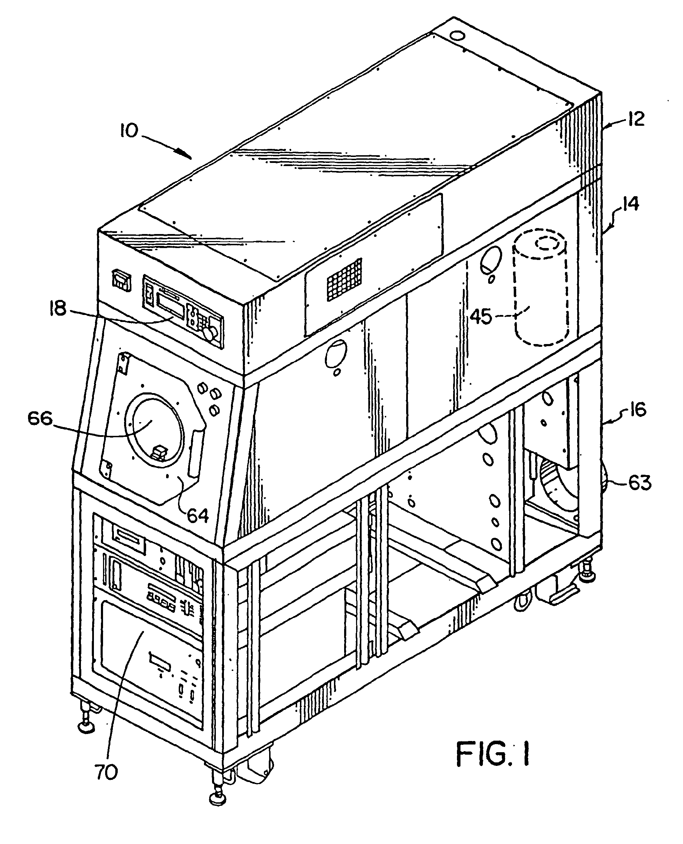

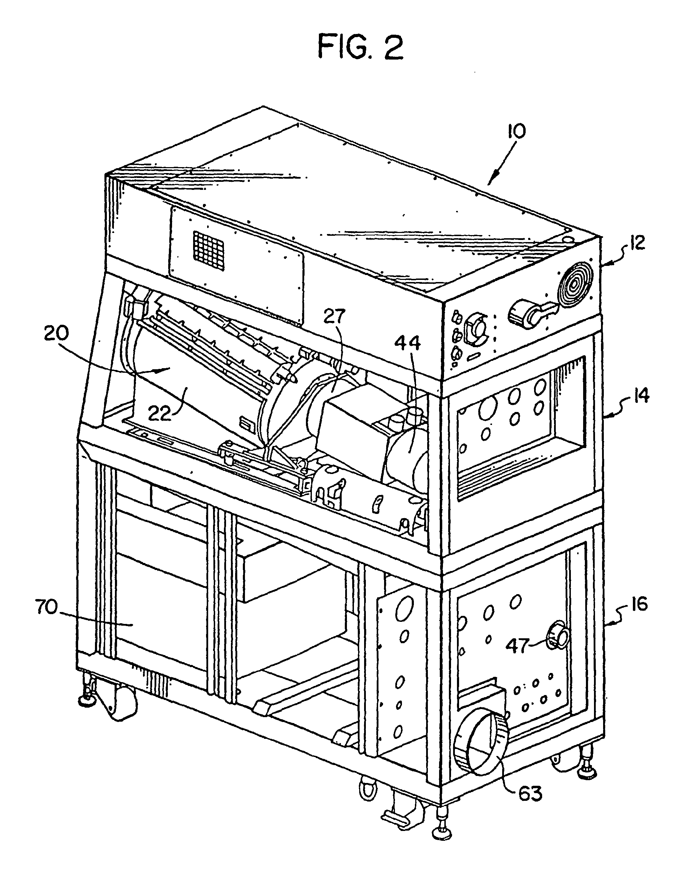

Turning now in detail to the drawings, as shown in Figs. 1 and 2, a workpiece processing system 10 preferably includes a first module12, a second modulel4, and a third module 16. More or less modules may be included in the processing system 10, but three modules are preferred. Each module houses a variety of processing system components.

The processing system 10 may be used to process workpieces of various sizes, but is typically configured to process workpieces of one size, such as 200 or 300 mm diameter semiconductor wafers. The area that the processing system 10 occupies on a clean room floor, or the system's "footprint," is preferably minimized to increase the amount of space available for additional processing systems and/or other clean room equipment. In the standalone processing system 10, which uses a modular construction to be as compact as possible, the size of the workpieces to be processed generally dictates the minimum possible size of the system footprint, as well as the minimum height of the system. Thus, with certain limitations, the smaller the size of the workpieces that are processed in the processing system 10, the smaller the system footprint and height may be.

When processing 200 mm semiconductor wafers, the footprint of the third module 16, or the bottom module, is preferably 46 to 60 cm wide by 115 to 135 cm long, more preferably 50 cm wide by 125 cm long. For processing larger workpieces, such as 300 mm semiconductor wafers, the third module 16 is preferably 60 cm to 70 cm wide by 115 cm to 135cm long, more preferably 65 cm wide by 125 cm long. The first and second modules 12, 14 preferably have similar cross-sectional areas to the third module 16, and may be slightly smaller, as illustrated in Figs. 1 and 2.

When configured for processing 200 mm semiconductor wafers, the first, second, and third modules 12, 14, 16 preferably have a combined height of 145 to 155 cm, more preferably 150 cm. For processing larger workpieces, such as 300 mm semiconductor wafers, the first, second, and third modules 12, 14, 16 preferably have a combined height of 155 cm to 175 cm, more preferably 160 cm. Accordingly, the footprint and height of the standalone workpiece processing system 10 are generally significantly smaller than those of typical existing processing systems. Thus, the processing system 10 is compact, and takes up relatively little space in a clean room environment.

The first module 12 preferably includes a system controller, which includes a control panel and display 18 at the front of the first module 12 for controlling and monitoring operation of the system. The first modulel2 also preferably includes a system power supply and any other electrical or electronic devices required for performing the various system operations.

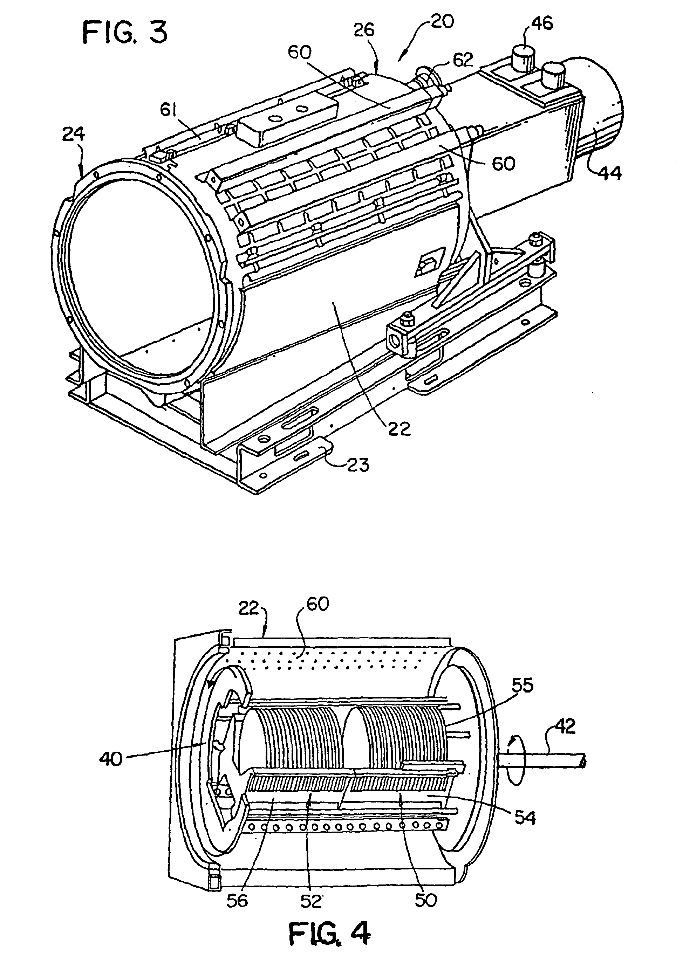

The second module 14 includes a processing chamber assembly 20, as illustrated in Figs. 2 and 3. The processing chamber assembly 20 includes a substantially cylindrical processing chamber 22 or bowl that is mounted to the second module 14 via support mounts 23. The support mounts 23 are preferably attached to support beams or another suitable base structure in the second module 14 via bolts or fasteners.

The second module 14 further includes a door 64 to provide access into the processing chamber 22. The door 64 preferably forms a seal with a front end 24 of the processing chamber 22. A window 66 is preferably located in the door 64 for allowing visual inspection into the processing chamber 22.

The processing chamber 22 may be oriented horizontally but is preferably inclined upwardly at an angle of, for example, 5-30°, and preferably about 10°, so that the front end 24 of the processing chamber 22 is at a higher elevation than the back end 26 of the processing chamber 22. Examples of such a processing chamber 22 and chamber assembly 20 are described in U.S. Patent No. 6,418,945.

A rotor 40, as illustrated in Fig. 4, is preferably rotatably supported within the processing chamber 22. A drive shaft 42 extends from the back of the rotor 40 into a motor 44 located at the back end 26 of the processing chamber 22. Power cables extending from the system controller in the first module 12 preferably provide electrical power and control to the motor 44 via connectors 46. The back end 26 of the processing chamber 22 is preferably sealed with a suitable seal assembly 27.

The rotor 40 preferably incorporates a dual-cassette design including a first or back cassette position 50, and a second or front cassette position 52. As a result, the rotor 40 can hold two carriers or cassettes, a first cassette 54, and a second cassette 56. Workpieces 55 are held within slots or wafer positions within each cassette 54, 56. Typically, the cassettes hold, for example, up to 25 wafers, although other cassette sizes may be used. The workpieces 55 are spaced apart from each other within the cassette, to allow processing fluids and/or gases to contact all surfaces of the workpieces 55.

The cassettes are generally standard components available from various manufacturers, although the size, shape, and features of different types of cassettes may vary. The rotor 40 may be adapted to hold a specific cassette (model number) from a specific manufacturer. Thus, the features and dimensions of the rotor 40 are adapted to the specific size, shape, and features of the cassettes selected for use in the processing system 10. Specific examples of rotors and cassettes that may be used in the processing chamber 22 are described in detail in U.S. Patent No. 6,418,945.

For ease of design, manufacture, and use, the first cassette 54 is preferably of the same design as the second cassette 56, so that the first and second cassettes positions 50, 52 within the rotor 40 may be the same. Although the invention contemplates any rotor having positions for first and second cassettes, regardless of whether the cassettes are of the same design, using two of the same cassettes: (a) allows the first and second cassette positions 50, 52 to be the same; (b) allows the rotor to be generally symmetrical; and (c) makes the loading sequence of the cassettes 54, 56 irrelevant.

Depending upon the chemicals to be used in the processing system 10, the rotor 40 and the processing chamber 22, as well as other components exposed to the chemicals, may be made of stainless steel, or alternatively the rotor and processing chamber material may be Teflon® (i.e., fluorine containing resins), or another suitable material. In a preferred embodiment, harsh chemicals, such as acids and solvents (e.g., HF, HCl, H₂SO₄, and H₂O₂), are not used in the processing system 10, so that a stainless steel processing chamber 22 and rotor 40 may be used, and so that any negative impact on the environment is substantially minimized.

As illustrated in Fig. 4, spray manifolds 60 for delivering processing fluid and/or rinse water preferably extend substantially along the entire length of the processing chamber 22. The manifolds 60 have spray nozzles or other openings directed into the processing chamber 22 for spraying liquids or gases into the processing chamber 22. A vent 62 is preferably included to exhaust gases or vapors from the processing chamber 22, as well as a drain 47 to remove liquids from the processing chamber 22.

The processing chamber 22 may further include various other components to enhance processing of the workpieces 55. For example, the processing chamber 22 may include: (a) an anti-static generator to reduce static electricity within the chamber 22; (b) one or more heaters to heat the workpieces 55 and/or the processing and or rinsing fluids; (c) an ozone destructor 45 to convert ozone into oxygen.

The third module 16 preferably serves as a process fluid storage compartment. The third module 16 preferably contains an ozone generator 70 in communication with or connecting with the processing chamber 22 for providing ozone gas into the processing chamber 22. The ozone generator 70 is preferably connected to a gas spray manifold 61 in the processing chamber 22 via one or more ozone delivery lines (not shown). In a preferred embodiment, the ozone generator 70 is a high capacity ozone generator that may generate up to 240g/cubic meter of ozone, or approximately 90gm/hour of ozone. If needed, separate cooling water lines may be routed to the ozone generator.

A DI water supply is preferably in communication with the processing chamber 22 for supplying DI water into the processing chamber 22. The DI water may be supplied from a DI water reservoir located in the third module 16, or may be supplied from an external source via one or more fluid delivery lines or other suitable fluid delivery means. One or more heaters may be located in the third module 16, or in another suitable location, for heating the DI water before it enters the processing chamber 22.

The third module 16 may house additional processing fluid supplies, such as an ammonium hydroxide (NH₄OH) supply, and/or any other suitable processing fluid supplies. Any fluid supplies used in the processing system 10 preferably communicate with the processing chamber 22 via one or more fluid delivery lines. A purge gas and/or drying gas (e.g., N₂) and/or clean dry air (CDA) ,if used, are typically supplied provided to the system from the fab or facility.

The third module 16 may further include pumps, filters, and/or other components for effectively providing the processing fluids and/or gases into the processing chamber 22. Additionally, the third module 16 may include alarms, sensors, and other monitoring devices to detect processing fluid levels in the processing chamber and to alert an operator when a problem may exist within the processing chamber. One or more of these devices may alternatively be located in the first or second modules 12, 14.

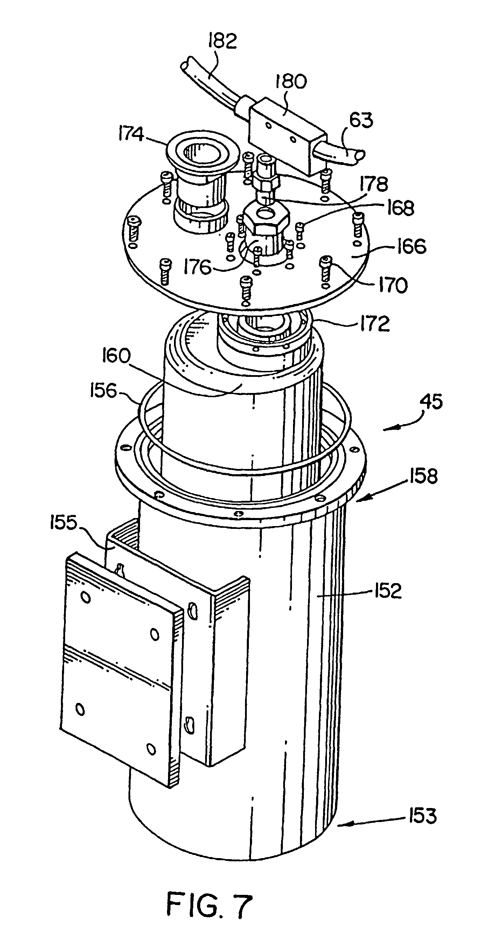

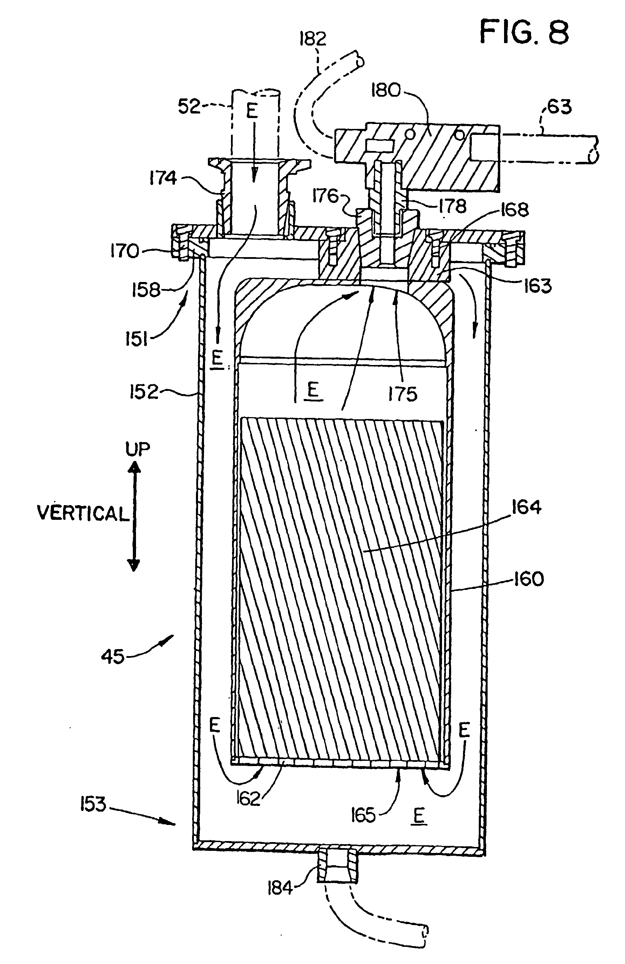

Referring to Figures 7 and 8, a preferred ozone destructor 45 has an upper end 151 and a lower end 153. The exhaust line 52 from the process chamber 22 connects at an inlet 174 at or near the upper end 151 of the ozone destructor 45. As shown with the arrows E in Figure 8, exhaust from the process chamber flows down within the ozone destructor 45, reverses direction, and then flows up through a catalyst 164 and then out of the ozone destructor 45 through an outlet to the system or enclosure exhaust line 63. The drain line is either valved or trapped to prevent ozone flow to the drain.

While Figure 8 shows the ozone destructor 45 in a vertically upright position, with exhaust gas flowing vertically up through the catalyst 164, the ozone destructor 45 may also have a different position or orientation, so long as there is a vertical component to the flow direction through the catalyst.

Referring still to Figure 8, by having the exhaust gas enter and exit at or adjacent to the top of the ozone destructor 45, and flow upwardly through the catalyst 164, the catalyst 164 is better isolated from the chamber exhaust line 52 and the process chambers. Consequently, potential for catalyst particles to move into the chamber exhaust line or process chambers is reduced, as catalyst particles would have to move against both gravity and exhaust flow to move into the chamber exhaust line 52. As exhaust also flows up through the catalyst, any condensation within the catalyst can run down, via gravity and drain out. This helps to prevent loss of catalytic action due to saturation of the catalyst. Ozone in exhaust flow moving through the catalyst 164 is converted into oxygen, which can then be vented out through the system exhaust 63.

Referring still to Figures 7 and 8, the following additional detailed description relates to a preferred ozone destructor design, without individually describing any single essential elements of the invention. The ozone destructor 45 has an outer container 152 which may be supported on or in an enclosure with a mounting bracket 155. A lid 166 is supported and attached to a flange 158 of the outer container 152. An o-ring or seal 156 seals the lid 166 to the outer container 152. Lid bolts or fasteners 170 secure the lid 166 to the outer container 152. An inner container or canister 160 contains the catalyst 164, typically a manganese dioxide-based catalyst suitable for decomposing ozone into oxygen. A perforated plate 162 at the lower end of the canister 160 holds the catalyst 164 (typically beads of solid material) within the canister 160. The perforated plate 162 has openings allowing exhaust gas to enter at the bottom, with the openings forming canister inlets 165. A canister collar 163 is attached at the top or upper end of the canister or inner container 160. Canister mounting bolts 168 attach the canister 160 via the canister collar 163 to the lid 166. A canister o-ring 172 seals the canister collar 163 against the lid 166. A lid bushing 176 extends through an opening in the lid and into the canister collar 163, connecting with the canister outlet 175, at the top or upper end of the canister 160. A pipe nipple 178 connects an aspirator 180 with the canister outlet 175 through the lid bushing 176. A clean dry air supply line 182 connects to the inlet side of the aspirator 180. The outlet side of the aspirator 180 connects, directly or indirectly to the system exhaust line 63. The aspirator 180 may be replaced by an air amplifier. The inlet 174 provides an entry flow path into the ozone destructor 45, through the lid 166.

Referring to Figure 8, the canister or inner container 160 containing the catalyst 164 is preferably suspended within the outer container 152. As shown in Figure 7, the outer container 152 and inner container 160 are preferably cylindrical, although other cross section shapes may also be used. Referring to Figure 8, an annular flow path extends from the inlet 174 down within the ozone destructor 45, between the outside walls of the inner container 160 and the inside walls of the outer container 152 to the canister inlets 165. Of course, other types of ozone destructors may also be used, including thermal, UV, and catalytic devices, or other equivalents which can convert, neutralize or destroy ozone.

In use, workpieces 55 are loaded into cassettes 54, 56 either manually or via a robot or other automated device. The door 64 on the second module 14 is opened, preferably manually by an operator, to provide access into the processing chamber 22. The first cassette 54 is lifted and placed into the rotor 40, as described in U.S. Patent No. 6,418,945. The first cassette 54 is moved toward the back of the rotor 40 until it can be moved no farther. As the rotor 40 is preferably positioned at an inclined angle, as shown in Figs. 2 and 3, the first cassette 54 moves down into and is seated into the first cassette position 50 within the rotor 40 with some assistance by gravity.

With the first cassette 54 installed within the rotor 40, the operator loads the second cassette 56 into the rotor 40, following the same procedure. The second cassette 56 is moved into the rotor 40 until it contacts the first cassette 50, such that it can be moved no farther toward the back of the rotor 40.

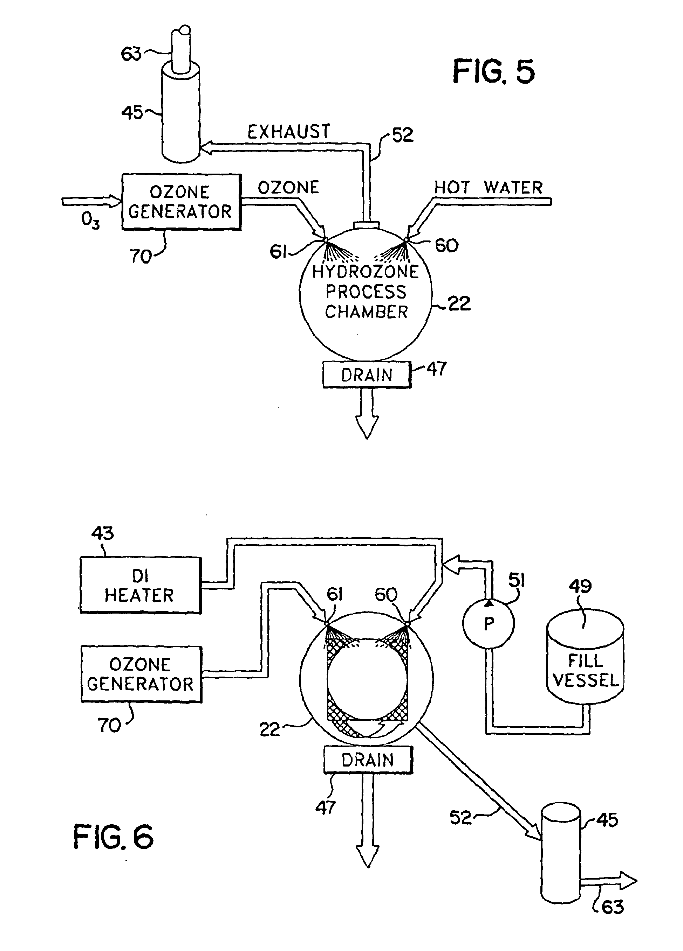

The operator then closes the door 64. A processing sequence can be preprogrammed into the system controller, or can be set up or selected by the operator using the control panel and display 18. In a typical application, as illustrated in the schematic diagram of Fig. 5, ozone gas is sprayed into the processing chamber 22 via manifolds 60 while the motor 44 spins the rotor 40. As the rotor 40 begins to rotate, the workpieces 55 are held within their respective cassettes 54, 56 by retainer bars or other retaining mechanisms, as described in U.S. Patent No. 6,418,945.

Heated DI water (provided by a heater 43 in the system or separately supplied) is concurrently sprayed into the processing chamber 22 in order to form a heated liquid boundary layer, through which the ozone gas may diffuse, on the surface of each of the workpieces 55. The DI water is preferably heated to a temperature of 30 to 110° C, more preferably 40, 50, 60, 70, 80 or 90 to 100° C. The ozone diffuses through the heated boundary layer, which accelerates diffusion-reaction kinetics, to react at the surface of the workpiece, as described in U.S. Patent No. 6,267,125, and U.S. Patent No. 6,497,768.

In an alternative embodiment, as illustrated in Fig. 6, ammonium hydroxide (NH₄OH) from a source or tank 49 may be mixed with (or injected via a pump 51) into heated DI water before the DI water enters the processing vessel 22, in order to enhance the cleaning process. The concentration of NH₄OH in the DI water is preferably very low, on the order of approximately 500-5000:1 or 1000-3000 or about 2000:1 parts DI water to NH₄OH. The addition of NH₄OH is particularly effective in photoresist removal applications, as it generally increases the removal rate of photoresist layers. NH₄OH mixed with heated DI water may also be supplied to the processing chamber as a separate step after first performing the steps of delivering ozone and DI water into the processing chamber 22. Use of ammonium hydroxide provides improved particle performance, cleaning efficiency of SiN particles, and removal of anti-reflective coatings.

After the cleaning and/or stripping steps are performed, the workpieces 55 are typically rinsed using DI water that is sprayed from the manifolds 60, and then dried with a drying gas, such as N₂ gas. A purge gas, such as N₂ gas, may be used between the rinsing and drying steps, or between other processing steps, to remove excess fluids from the processing chamber. Exhaust vapors and gases flow out of the chamber through the exhaust port 62 and into the ozone destructor 45. Ozone in the ozone destructor is converted in oxygen gas which flows out of the system enclosure via an exhaust gas duct 63, along with other exhaust gases or vapors. The various processing steps may be repeated one or more times to enhance the cleaning or stripping processes, as desired.

Referring to Figure 8, the exhaust gas moves into the ozone destructor at or near the upper end 151 and moves downwardly, as shown by the arrows E. The exhaust flow E moves to the lower end 153 of the ozone destructor 45, reverses direction, and flows upwardly through the perforated plate 162 and into the canister 160. As the exhaust flows through the catalyst 164 within the canister 160, ozone within the exhaust gas E is converted into oxygen. The aspirator or air amplifier 180 helps to draw exhaust gas flow through the ozone destructor 45, thereby eliminating any back pressure on the process chamber. Oxygen converted by the catalyst 164, and any other exhaust gas components, move upwardly through the canister outlet 175, through the aspirator 180, and out via the system exhaust line 63.

If water vapor condenses within the catalyst 164, the liquid water drains out downwardly through the perforated plate 162 and collects at the lower end 153 of the outer container 152, where it can be removed by the liquid drain line 184. Consequently, saturation of the catalyst 164 with condensation is avoided. As the exhaust flow inlet into the ozone destructor 45 is at or near the upper end 151, the potential for catalyst particles getting into the chamber exhaust line 52 or the process chambers is greatly reduced. Similarly, the potential for condensed liquid to block the exhaust line 52 is reduced or eliminated. Preferably, the components of the ozone destructor 50 coming in contact with exhaust gas are made of stainless steel having a PFA coating to better resist corrosion by chemical vapors in the exhaust flow.

In general, when processing 200 mm workpieces, the processing system 10 has a throughput of approximately 200 workpieces/hour. When processing 300 mm workpieces, the processing system 10 has a throughput of approximately 100 workpieces/hour. The actual throughput will depend on the type of workpiece-processing application that is performed, the number of workpieces processed at one time, and the number of processing steps that are repeated.

The processing system 10 and methods described herein may be used in several different workpiece-processing applications, such as the following: (1) post-ash cleaning; (2) photoresist stripping; (3) organic material cleaning (4) photo reworking/reclaiming; (5) post-etch cleaning; and any other suitable processing applications.

The processing system 10 provides several advantages over existing processing systems. First, the system 10 is extremely compact so it does not take up significant space in a clean room environment. Due to the limited number of components and processing stations required, the processing system 10 is also relatively inexpensive. The processing system 10 uses relatively mild processing chemicals, such as ozone gas and DI water, so there is minimal, if any, negative impact on the environment. Additionally, larger batches of workpieces can be processed in a relatively short amount of time in the processing system 10.

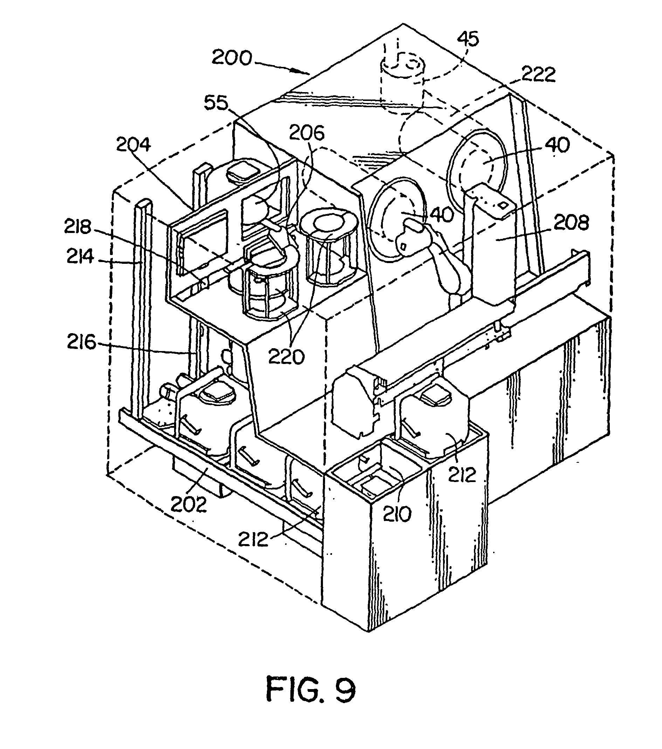

The ozone destroyer or converter 45 shown in Figs. 7 and 8 may also be used in other types of systems performing processing with ozone. For example, the converter 45 may be used in an automated or robotic system, as described in International Patent Application Publication No. WO 02/05313 A2 and as shown in Fig. 9. In this type of automated system, the ozone destroyer 45 is connected to the gas exhaust from the ozone process chamber 22. The process chamber may include a rotor adapted to receive wafers directly, or to receive carriers 220 or cassettes. This system 200 has an indexer 202, preferably at a first elevation ; a docking station 204, preferably at a second elevation higher than the first elevation; a transfer station 206 adjacent to the docking station; an ozone process station or chamber 22 or 222; and a robot 208 movable between the transfer station and the process chamber. This type of system may also include a loader 210 associated with the indexer, the loader having a load elevator for moving a closed container or FOUP 212of workpieces or wafers between an up position, and a down position, and with the load elevator in the down position substantially aligned with the indexer at the first elevation. This type of system may also have at least one docking station elevator 214, 216 for moving a container vertically from the first elevation to the docking station at the second elevation; and a container door remover 218 at the docking station. These features, as described in WO 02/05313 A2, may be used as a system, in combination with the ozone converter 45.

## Claims

1. A system (10) for processing one or more workpieces (55), the system (10) having a process chamber (22) within an enclosure and a rotor (40) rotatable in the process chamber (22) for holding the workpieces (55),
at least one spray manifold (60) in the process chamber (22) for spraying a process liquid including de-ionized water onto the workpieces (55);
a heater (43) for heating the process liquid; a chamber exhaust line (52) connecting from the process chamber (22) to the inlet (174) of the ozone destructor (45); and with a ozone destructor (45) suitable for converting ozone in exhaust gases flowing out of the process chamber (22) into oxygen. **characterized by**:
an ozone gas source (70) within the enclosure for supplying ozone gas into the process chamber (22);
the ozone destructor (45) within the enclosure having an inlet (174) and an outlet (175);
a system exhaust line (63) connecting to the outlet (175) of the ozone destructor (45) and extending out of the enclosure.

2. The system (10) of claim 1, further comprising:
a third module (16) including a processing fluid supply comprising an ozone generator (70) for supplying ozone gas into the process chamber (22);
a second module (14) on top of and attached to the third module (16) and including the process chamber (22), the processing fluid supply being in communication with the process chamber (22);
a first module (12) on top of and attached to the second module (14);
and the system having a footprint of 0.5 to 1.1 square meters.

3. The system (10) of claim 1, the ozone gas source (70) producing ozone gas at a rate of at least 90 grams/hour.

4. The system (10) of claim 1, wherein the system (10) is used as a subsystem in an automated workpiece processing system (200) also having:
a workpiece container holding area (202);
a docking station (204) for docking and opening workpiece containers;
a transfer station (206) adjacent to the docking station (204); and
a robot (208) moveable between the transfer station (206) and one or more process chambers (22, 222), to move workpieces (55) between them.

5. A method for processing one or more workpieces (55) with ozone in a wafer processing system (10) within an enclosure, by loading the workpieces (55) into a process chamber (22), rotating the workpieces (55) and applying heated water to the workpieces (55), comprising:
loading a first cassette of workpieces (55) into a rotor (40) in the process chamber (22);
loading a second cassette of workpieces (55) into the rotor;
heating water to 30-95°C;
spraying the heated water onto the rotating workpieces (55);
introducing ozone gas from an ozone gas source (70) within the enclosure to the process chamber (22), the ozone gas diffusing through a layer of the heated water on the workpieces (55) ;
purging the ozone gas out of the process chamber (22) and into an ozone destructor (45) within the enclosure; converting the ozone gas into oxygen in the ozone destructor (45); and
drying the workpieces (55).

## Patentansprüche

1. System (10) zum Bearbeiten eines oder mehrerer Werkstücke (55), wobei das System aufweist eine Prozesskammer (22) innerhalb eines Gehäuses und einen Rotor (40), der zum Halten der Werkstücke (55) in der Prozesskammer (22) rotieren kann und mindestens einen Sprühverteiler (60) in der Prozesskammer (22) zum Sprühen einer Prozessflüssigkeit mit deionisiertem Wasser auf das Werkstück (55),
eine Heizvorrichtung (43) zum Erwärmen der Prozessflüssigkeit,
eine Kammerabgasleitung (52), die die Prozesskammer (22) mit dem Einlass (174) des Ozonvernichters (45) verbindet,
einem Ozonvernichter (45), der geeignet ist zum Umwandeln von Ozon, das sich in Abgasen befindet, welche aus der Prozesskammer (22) kommen, in Sauerstoff,
**gekennzeichnet durch**:
eine Ozongasquelle (70) innerhalb des Gehäuses zum Zuführen von Ozongas in die Prozesskammer (22),
den Ozonvernichter (45) innerhalb des Gehäuses, der einen Einlass (174) und einen Auslass (175) hat, und
eine Systemabgasleitung (63), die mit dem Ausgang (175) des Ozonvernichters (45) verbunden ist und aus dem Gehäuse herausragt.

2. System (10) gemäß Anspruch 1, ferner aufweisend ein drittes Modul (16) mit einer Prozessflüssigkeitsversorgung, das einen Ozongenerator (70) zum Versorgen der Prozesskammer (22) mit Ozongas aufweist,
ein zweites Modul (14) oberhalb des dritten Moduls (16) und verbunden mit dem dritten Modul (16) und das die Prozesskammer (22) enthält, wobei die Prozessflüssigkeitsversorgung mit der Prozesskammer (22) verbunden ist;
ein erstes Modul (12) oberhalb des zweiten Moduls (14) und verbunden mit dem zweiten Modul (14),
wobei das System eine Grundfläche von 0,5 bis 1,1 Quadratmeter hat.

3. System (10) gemäß Anspruch 1, wobei die Ozongasquelle (70) Ozongas mit einer Rate von wenigsten 90 Gramm/Stunde produziert.

4. System (10) gemäß Anspruch 1, wobei das System (10) als ein Teilsystem in einem automatisierten Werkstückbearbeitungssystem (200) benutzt wird, welches weiterhin aufweist:
einen Werkstückbehälterhaltebereich (202),
eine Kopplungsstation (204) zum Ankoppeln und Öffnen von Werkstückbehältern,
eine Übergabestation (206), die an die Kopplungsstation (204) angrenzt, und
einen Roboter (208), der zwischen der Übergabestation (206) und einer oder mehreren Prozesskammern (22, 222) bewegbar ist, um Werkstücke (55) zwischen diesen zu bewegen.

5. Verfahren zum Bearbeiten von einem oder mehreren Werkstücken (55) mit Ozon in einem Wafer-Herstellungs-System (10) in einem Gehäuse, wobei die Werkstücke (55) in eine Prozesskammer (22) eingeladen werden, die Werkstücke (55) rotiert werden und erhitztes Wasser auf die Werkstücke (55) aufgebracht wird, aufweisend:
Laden einer ersten Kassette von Werkstücken (55) in einen Rotor (40) in der Prozesskammer (22),
Laden einer zweiten Kassette von Werkstücken (55) in den Rotor,
Erhitzen des Wassers auf 30 - 95 Grad Celsius;
Sprühen des aufgeheizten Wassers auf die rotierenden Werkstücke (55),
Einleiten von Ozongas von einer Ozongasquelle (70) innerhalb des Gehäuses in die Prozesskammer (22), wobei das Ozongas durch eine Schicht des aufgeheizten Wassers auf den Werkstücken (55) hindurch diffundiert;
Entleeren des Ozongases aus der Prozesskammer (22) in einen Ozonvernichter (45) innerhalb des Gehäuses,
Umwandeln des Ozongases in Sauerstoff im Ozonvernichter (45) und Trocknen der Werkstücke (55).

## Revendications

1. Système (10) pour traiter une ou plusieurs pièces à usiner (55), le système (10) comportant une chambre de traitement (22) à l'intérieur d'une enceinte et un rotor (40) monté rotatif dans la chambre de traitement (22) pour maintenir les pièces à usiner (55),
au moins un distributeur de pulvérisation (60) dans la chambre de traitement (22) pour pulvériser un liquide de traitement comprenant de l'eau déminéralisée sur les pièces à usiner (55) ;
un dispositif de chauffage (43) destiné à chauffer le liquide de traitement;
une conduite d'échappement de chambre (52) connectée de la chambre de traitement (22) à l'entrée (174) du destructeur d'ozone (45) ; et
avec un destructeur d'ozone (45) approprié pour convertir de l'ozone présent dans des gaz d'échappement sortant de la chambre de traitement (22) en oxygène, **caractérisé par** :
une source d'ozone gazeux (70) à l'intérieur de l'enceinte pour alimenter en ozone gazeux la chambre de traitement (22) ;
le destructeur d'ozone (45) à l'intérieur de l'enceinte ayant une entrée (174) et une sortie (175) ;
une conduite d'échappement de système (63) connectée à la sortie (175) du destructeur d'ozone (45) et s'étendant hors de l'enceinte.

2. Système (10) selon la revendication 1, comprenant en outre :
un troisième module (16) comprenant une alimentation en fluide de traitement comprenant un générateur d'ozone (70) pour alimenter en ozone gazeux la chambre de traitement (22) ;
un deuxième module (14) au-dessus de et fixé sur le troisième module (16) et comprenant la chambre de traitement (22), l'alimentation en fluide de traitement étant en communication avec la chambre de traitement (22) ;
un premier module (12) au-dessus de et fixé sur le deuxième module (14) ; et le système ayant une surface de 0,5 à 1,1 mètre carré.

3. Système (10) selon la revendication 1, dans lequel la source d'ozone gazeux (70) produit de l'ozone gazeux à un débit d'au moins 90 grammes/heure.

4. Système (10) selon la revendication 1, dans lequel le système (10) est utilisé comme sous-système dans un système de traitement de pièces à usiner automatisé (200) comportant également :
une zone contenant des conteneurs de pièces à usiner (202) ;
un poste d'accostage (204) destiné à accoster et ouvrir les conteneurs de pièces à usiner ;
un poste de transfert (206) adjacent au poste d'accostage (204) ; et
un robot (208) mobile entre le poste de transfert (206) et une ou plusieurs chambres de traitement (22, 222), pour déplacer les pièces à usiner (55) entre eux.

5. Procédé pour traiter une ou plusieurs pièces à usiner (55) avec de l'ozone dans un système de traitement de tranches (10) à l'intérieur d'une enceinte, en chargeant les pièces à usiner (55) dans une chambre de traitement (22), mettant en rotation les pièces à usiner (55) et appliquant de l'eau chauffée aux pièces à usiner (55), comprenant les étapes consistant à :
charger une première cassette de pièces à usiner (55) dans un rotor (40) dans la chambre de traitement (22) ;
charger une seconde cassette de pièces à usiner (55) dans le rotor ;
chauffer de l'eau à 30 à 95 °C ;
pulvériser l'eau chauffée sur les pièces à usiner en rotation (55) ;
introduire de l'ozone gazeux à partir d'une source d'ozone gazeux (70) à l'intérieur de l'enceinte dans la chambre de traitement (22), l'ozone gazeux diffusant à travers une couche de l'eau chauffée sur les pièces à usiner (55) ;
extraire l'ozone gazeux par purge de la chambre de traitement (22) et dans un destructeur d'ozone (45) à l'intérieur de l'enceinte ;
convertir l'ozone gazeux en oxygène dans le destructeur d'ozone (45) ; et
sécher les pièces à usiner (55).
